# EUROPÄISCHE PATENTANMELDUNG

(11) **EP 4 190 307 A1**
(43) Veröffentlichungstag der Anmeldung: **07.06.2023**
(21) Anmeldenummer: 22206275.4
(22) Anmeldetag: 09.11.2022
(51) Int. Cl.: A61K 8/37, A61K 8/92, A61Q 5/00, A61Q 5/06

(54) **KOSMETISCHE LEAVE-ON-PRODUKTE ZUR VERBESSERUNG DER FRISIEREIGENSCHAFTEN**

(30) Priorität: 25.11.2021 DE 102021213248
(71) Anmelder: Henkel AG & Co. KGaA, 40589 Düsseldorf (DE)
(72) Erfinder: Battermann, Marlene, 21271 Asendorf (DE); Kerl, Sylvia, 25474 Bönningstedt (DE)

(57) **Zusammenfassung**

Haarbehandlungsmittel, die in einem kosmetischen Träger 3-Hydroxypropyloctansäureester (Propandiol Caprylate) und mindestens ein Wachs enthalten, eignen sich als Leave-on Produkte für die Verbesserung der Frisierbarkeit von Haaren.

## Beschreibung

Die vorliegende Anmeldung beschreibt ein Haarbehandlungsmittel, welches in einem kosmetischen Träger Propandiol Caprylate und mindestens ein Wachs umfasst, sowie dessen Verwendung. Außerdem wird ein Verfahren zur Haarbehandlung unter Einsatz des Haarbehandlungsmittels offenbart.

Nicht zuletzt durch die starke Beanspruchung der Haare wie Färben oder Dauerwellen, häufiges Reinigen mit Shampoos und anschließendes Trocknen unter Föhnhitze sowie durch Umweltbelastungen nimmt die Bedeutung von Haarpflegeprodukten stetig zu.

Gleichzeitig rückt das Thema Nachhaltigkeit, geprägt durch ein stetig wachsendes diesbezügliches Bedürfnis der Verbraucher, zunehmend in den Fokus der Kosmetikhersteller.

Neben gut biologisch abbaubaren Inhaltsstoffen besteht in Zeiten wachsender Wasserknappheit zusätzlich der Wunsch nach Produkten, deren Anwendung nicht an einen erhöhten Wasserverbrauch gekoppelt ist. Beim Abspülvorgang eines herkömmlichen Rinse-off-Haarpflegeproduktes werden bei üblichem Gebrauch unter der Dusche beispielsweise mehrere Liter Wasser verbraucht.

Leave-in-Haarpflegeprodukte wie Haarmasken oder Haarkuren sind an sich bekannt und im Handel erhältlich. Ein Großteil dieser Produkte umfasst Silikone, denn diese verleihen Haaren mannigfaltige Konditionierungsvorteile.

Bei der Bereitstellung nachhaltiger Produkte wird weitestgehend auf die Verwendung von Silikonen verzichtet, doch führt dies meist zu Nachteilen in der Pflegeperformance der Produkte.

Darüber hinaus ist insbesondere bei der regelmäßigen Anwendung von Leave-in-Produkten dafür Sorge zu tragen, dass kein beschwerender Builtup-Effekt auftritt, der den Haaren ein unästhetisches Aussehen verleiht.

Neben Haarpflegeprodukten spielen Haarbehandlungsmittel, die einer permanenten oder temporären Formgebung der Haare (Styling) dienen, in der Kosmetik eine wichtige Rolle. Im Stand der Technik sind deshalb zahlreiche kosmetische Zusammensetzungen bekannt, wie zum Beispiel Haarsprays, Haarwachse, Haargele, Haarschäume, Pomaden, Stylingpulver, Stylingclays, Haarlotionen, Combing Creams etc.

Diese Styling- oder Frisierprodukte unterscheiden sich nicht nur in der Aufbringung auf dem Haar (Aufsprühen, Aufbringen mit der Hand, Aufbringen mit einem Kamm, etc.), sondern insbesondere in ihrer Konsistenz und ihrem Zweck (Stylingergebnis). So dienen Haarsprays insbesondere dazu, eine fertig gestylte Frisur haltbarer zu machen. Pomaden wurden in erster Linie entwickelt, um Haar auf eine glatte, saubere Weise zu präsentieren und gleichzeitig ein hochglänzendes Finish zu erzielen. Stylingcremes (auch Haarlotionen genannt) haben eine Lotions-artige Konsistenz (im Gegensatz zu der festen, pastösen Konsistenz von Pomaden und Pasten) und können durch ihre Feuchtigkeitsspendenden Eigenschaften insbesondere lockiges/krauses Haar kontrollieren und pflegen. Stylingcremes bieten einen leichten, weicheren Halt im Vergleich zu allen anderen Haarstylingprodukten.

Viele Verbraucher wünschen sich ein natürlich aussehendes Stylingergebnis, bei dem sich das Haar nicht klebrig oder fest anfühlt, sondern geschmeidig und gepflegt.

Zur Erzielung eines natürlichen Stylingergebnisses bei gewünschter Halteleistung (beispielsweise niedrig oder mittel) wird regelmäßig eine Kombination aus synthetischen Polymeren und Wachsen eingesetzt. Bei der Bereitstellung nachhaltiger Produkte wird weitestgehend auf die Verwendung von synthetischen Polymeren verzichtet, doch führt dies meist zu Nachteilen in der Stylingperformance der Produkte.

Darüber hinaus kann die Einarbeitung von Wachsen insbesondere bei der Kombination mit anderen kosmetischen Inhaltsstoffen problematisch sein.

Es besteht weiterhin Bedarf nach natürlicheren kosmetischen Produkten mit sehr gutem Styling- und Pflegevermögen, die damit behandelten Haaren eine hervorragende Frisierbarkeit sowie ein gepflegtes, nicht klebriges oder steifes Haargefühl verleihen.

Diese Aufgabe wird gelöst durch ein Haarbehandlungsmittel, das in einem kosmetischen Träger
a) 3-Hydroxypropyloctansäureester (Propandiol Caprylate) und
b) mindestens ein Wachs enthält.

Dieses neuartige Haarbehandlungsmittel gewährleistet eine ausgezeichnete Balance zwischen Frisierbarkeit, Styling und Haarpflege, insbesondere bei der Verwendung zur Behandlung geschädigter, spröder, stumpfer und/oder lockiger Haare in Form von Leave-on Produkten.

Die neue kosmetische Zusammensetzung umfasst zudem einen überwiegenden Anteil von Wirkstoffen aus nachhaltigen Quellen und ist stabil.

In einer bevorzugten Ausführungsform wird das erfindungsgemäße Haarbehandlungsmittel in Form einer Leave-on-Zusammensetzung bereitgestellt.

Unter einem kosmetischen Träger wird im Sinne der vorliegenden Erfindung ein wässriger, ein alkoholischer oder ein wässrig-alkoholischer Träger verstanden. Zum Zwecke der gleichzeitigen Pflege und Frisurgebung sind unter solchen Trägern beispielsweise Cremes, Emulsionen, Gele, Pasten zu verstehen, aber auch Tenside enthaltende, schäumende Lösungen wie beispielsweise Shampoos, Schaumaerosole, Schaumformulierungen oder andere Zubereitungen, die für die Anwendung auf dem Haar geeignet sind.

Als ersten wesentlichen Inhaltsstoff enthalten die erfindungsgemäßen Haarbehandlungsmittel 3-Hydroxypropyloctansäureester (Propandiol Caprylate).

Propanediol Caprylate ist als hochwirksamer Antischuppenwirkstoff bekannt, der ausschließlich aus natürlichen Quellen, beispielsweise durch Hydrolyse von Palmkernöl, anschließender Fermentation und Destillation der Hydrolyseprodukte sowie Veresterung von 1,3-Propandiol mit Caprylsäure erhältlich ist.

Propanediol Caprylate wirkt gezielt gegen Kopfschuppen verursachende und/oder verstärkende Malassezia-Stämme und kann als natürliche Alternative zu üblichen Antimykotika (wie Ketoconazol), Keratolytika (wie Salicylsäure) und/oder Keratostatika (wie Zink-Pyrithion oder Octopirox) in kosmetischen Haarbehandlungsmitteln verwendet werden.

Darüber hinaus wurden schaumstabilisierende Eigenschaften von Propanediol Caprylate in üblichen, Tenside enthaltenden Produkten beschrieben.

In den erfindungsgemäßen Haarbehandlungsmitteln, insbesondere in erfindungsgemäßen Haarbehandlungsmitteln, die als leave-on-Produkte konfektioniert werden, führt die Anwesenheit von Propanediol Caprylate in Kombination mit Wachsen zu einer(m) verbesserten Frisierbarkeit, Haargriff und zu erhöhter Geschmeidigkeit von Haaren.

Propanediol Caprylate ist im Handel erhältlich, beispielsweise von der Firma Symrise unter der Handelsbezeichnung Crinipan^{®} PMC green.

3-Hydroxypropyloctansäureester (INCI-Bezeichnung: Propanediol Caprylate) wird in den erfindungsgemäßen Haarbehandlungsmitteln bevorzugt in einer Menge von 0,01 bis 5 Gew.-%, mehr bevorzugt 0,05 bis 2 Gew.-% und besonders bevorzugt 0,1 bis 1 Gew.-% eingesetzt, wobei sich die Mengenangaben auf das Gesamtgewicht der Haarbehandlungsmittel beziehen.

Als zweiten wesentlichen Inhaltsstoff enthalten die erfindungsgemäßen Haarbehandlungsmittel mindestens ein Wachs, vorzugsweise ein natürliches Wachs.

Unter natürlichen Wachsen werden im Sinne der vorliegenden Erfindung pflanzliche Wachse, tierische Wachse, Mineralwachse oder Mischungen davon verstanden. Beispiele für Wachse aus diesen Gruppen sind beispielsweise Candelillawachs, Carnaubawachs, Espartograswachs, Guarumawachs, Japanwachs, Korkwachs, Reiskeimölwachs, Lorbeerbaumwachs, Kapokwachs, Baumwollwachs, Zuckerrohrwachs, Ouricurywachs, Sonnenblumen(kern)wachs, Jojobawachs, Rapswachs, Sojawachs, Reiswachs, Rosenblütenwachs, Fruchtwachs, Myrica Fruchtwachs, Bienenwachs und andere Insektenwachse, Walrat, Wollwachs (Lanolin), Bürzelfett, Shea Butter, Kakaobutter, weiterhin Mineralwachse, wie Ceresin und Ozokerit oder petrochemische Wachse, wie Petrolatum, Paraffinwachs, Isoparaffinwachs, Mikrowachs aus Polyethylen oder Polypropylen und Polyethylenglycolwachs (POE-Wachse), insbesondere POE-Lanolinalkoholether, POE-Lanolinalkoholacetat, POE-Cholesterinether, Lanolinfettsäurepolyethylenglykol oder hydrierter POE-Lanolinalkoholether. Es ist auch möglich, hydrierte oder gehärtete Wachse einzusetzen.

Vorzugsweise werden in den erfindungsgemäßen Haarbehandlungsmitteln Wachse aus den Gruppen der pflanzlichen und tierischen Wachse eingesetzt. Besonders bevorzugt sind Candelillawachs, Carnaubawachs, Espartograswachs, Korkwachs, Zuckerrohrwachs, Ouricurywachs, Sonnenblumen(kern)wachs, Jojobawachs, Rapswachs, Sojawachs, Reiswachs, Rosenblütenwachs, Fruchtwachs (wie Beerenwachs, Apfel(schalen)wachs, Orangen(schalen)wachs), Myrica Fruchtwachs, Bienenwachs, Insektenwachs, Walrat, Wollwachs, Bürzelfett, Shea Butter, Kakaobutter oder Mischungen davon.

Insbesondere bevorzugt werden in den erfindungsgemäßen Haarbehandlungsmitteln die folgenden Wachse eingesetzt: Candelillawachs (Euphorbia Cerifera (Candelilla) Wax), Carnaubawachs (Copernicia Cerifera (Carnauba) Wax), Beerenwachs (Rhus Verniciflua Peel Cera), Apfel(schalen)wachs (Pyrus Malus (Apple Peel) Wax), Orangen(schalen)wachs (Citrus Aurantium Dulcis Peel Wax) oder Mischungen davon. Es wurde gefunden, dass insbesondere die Kombination mindestens eines dieser Wachse mit Propandiol Caprylate (insbesondere) in (Leave-on)-Haarbehandlungsmitteln zur signifikanten Verbesserung der Frisiereigenschaften von Haaren führt.

Das oder jedes Wachs wird in den erfindungsgemäßen Haarbehandlungsmitteln bevorzugt in einer Menge von 0,001 bis 20 Gew.-%, mehr bevorzugt 0,005 bis 15 Gew.-% und besonders bevorzugt 0,01 bis 10 Gew.-% eingesetzt, wobei sich die Mengenangaben auf das Gesamtgewicht der Haarbehandlungsmittel beziehen.

Die Wirksamkeit der erfindungsgemäßen Haarbehandlungsmittel kann durch die zusätzliche Anwesenheit natürlicher Verdickungsmittel noch weiter gesteigert werden.

Unter natürlichen Verdickungsmitteln werden im Sinne der vorliegenden Erfindung vorzugsweise natürliche Hydrocolloide verstanden, denn es wurde gefunden, dass sie die haarpflegenden Eigenschaften der Wirkstoffmischung a) + b) verstärken können. Unter erfindungsgemäß geeigneten natürlichen Verdickungsmitteln werden beispielsweise Polysaccharide wie Glucanan, modifizierte und nicht-modifizierte Stärken, Amylose, Amylopektin, Dextrane, Cellulose und deren Derivate (Methylcellulose, Hydroxyalkylcellulose, Ethylhydroxyethylcellulose und Carboxymethylcellulose), Mannane, Xylane, Lignine, Chitin, Chitosane, Pektine, Alginsäuren und Alginate, Arabinogalactane, Carageenane und Carrageenate, Agar, Gumme (arabicum, karaya), Johannisbrotkernmehl, Galactomannane wie Guar-Gummi und dessen nicht-ionische Derivate (Hydroxypropyl Guar), Xanthangum, Caesalpinia Spinosa Gum (Tara Gum), Scleroglucan sowie Gemische davon verstanden, wobei modifizierte und nicht-modifizierte Stärken (wie beispielsweise Hydroxypropyl Starch Phosphate), Xanthan Gum, Caesalpinia Spinosa Gum (Tara Gum) oder Mischungen davon bevorzugt sind.

Insbesondere bevorzugt aufgrund ihrer den Haarglanz verstärkenden Wirkung sind natürliche Verdickungsmittel aus der Gruppe der Gumme und insbesondere bevorzugt Xanthan Gum, Caesalpinia Spinosa Gum (Tara Gum) oder Mischungen davon.

Das oder jedes natürliche Verdickungsmittel wird in den erfindungsgemäßen Haarbehandlungsmitteln bevorzugt in einer Menge von 0,001 bis 10 Gew.-%, mehr bevorzugt 0,005 bis 7,5 Gew.-% und besonders bevorzugt 0,01 bis 5 Gew.-% eingesetzt, wobei sich die Mengenangaben auf das Gesamtgewicht der Haarbehandlungsmittel beziehen.

Eine bevorzugte Ausführungsform der erfindungsgemäßen Haarbehandlungsmittel ist dadurch gekennzeichnet, dass sie - bezogen auf ihr Gesamtgewicht -
a) 0,01 - 5 Gew.-% Hydroxypropyloctansäureester (Propandiol Caprylate) und
b) 0,001 - 20 Gew.-% Candelillawachs (Euphorbia Cerifera (Candelilla) Wax), Carnaubawachs (Copernicia Cerifera (Carnauba) Wax), Beerenwachs (Rhus Verniciflua Peel Cera), Apfel(schalen)wachs (Pyrus Malus (Apple Peel) Wax), Orangen(schalen)wachs (Citrus Aurantium Dulcis Peel Wax) oder Mischungen davon enthalten.

Innerhalb dieser Ausführungsform sind erfindungsgemäße Haarbehandlungsmittel besonders bevorzugt, die Hydroxypropyloctansäureester (Propandiol Caprylate) und Carnaubawachs (Copernicia Cerifera (Carnauba) Wax) oder Hydroxypropyloctansäureester (Propandiol Caprylate) und Beerenwachs (Rhus Verniciflua Peel Cera) in den zuvor genannten Mengen enthalten.

Eine weitere bevorzugte Ausführungsform der erfindungsgemäßen Haarbehandlungsmittel ist dadurch gekennzeichnet, dass sie - bezogen auf ihr Gesamtgewicht -
a) 0,01 - 5 Gew.-% Hydroxypropyloctansäureester (Propandiol Caprylate),
b) 0,001 - 20 Gew.-% Candelillawachs (Euphorbia Cerifera (Candelilla) Wax), Carnaubawachs (Copernicia Cerifera (Carnauba) Wax), Beerenwachs (Rhus Verniciflua Peel Cera), Apfel(schalen)wachs (Pyrus Malus (Apple Peel) Wax), Orangen(schalen)wachs (Citrus Aurantium Dulcis Peel Wax) oder Mischungen davon, und
c) 0,001 - 10 Gew.-% Caesalpinia Spinosa Gum, Xanthan Gum oder Mischungen davon enthalten.

Neben den zuvor genannten wesentlichen und bevorzugten weiteren Komponenten können die erfindungsgemäßen Haarbehandlungsmittel weitere Inhaltsstoffe umfassen, die ihnen vorteilhafte Eigenschaften verleihen.

Als weitere Komponenten können beispielsweise eingesetzt werden:
- pflanzliche Öle,
- Filmbildner,
- Polyole,
- Fettalkohole,
- kationische Tenside,
- Trimethylglycin
- oder Mischungen davon.

Geeignete pflanzliche Öle können aus Vitis Vinifera (Trauben)-Kernöl, Cocos Nucifera (Kokosnuss)-Öl, Prunus Amygdalus Dulcis (Süßmandel)-Öl, Juglans Regia (Walnuss)-Kernöl, Prunus Persica (Pfirsich)-Kernöl, Persea Gratissima (Avocado)-Öl, Melaleuca Alternifolia (Teebaum)-Blattöl, Glycin Soja (Sojabohnen)-Öl, Gossypium Herbaceum (Baumwoll)-Samenöl, Sesamum Indicum (Sesam)-Samenöl, Helianthus Annuus (Sonnenblumen)-Samenöl, Camellia Japonica Samenöl (Tsubakiöl), Oenothera Biennis (Nachtkerzen)-Öl, Oryza Sativa (Reis)-Kleieöl, Elaeis Guineensis (Palm)-Öl, Mangifera Indica (Mango)-Kernöl, Vaccinium Macrocarpon (Cranberry)-Kernöl, Hippophae Rhamnoides Fruchtöl, Wiesenschaumkraut-Öl, Carthamus Tinctorius (Saflor)-Samenöl, Macadamia Ternifolia Kernöl, Amaranthus Spinosus Samenöl, Argania Spinosa Kernöl, Bambusöl, Olea Europaea (Oliven)-Fruchtöl, Triticum Vulgare (Weizen)-Keimöl, Cucurbita Pepo (Kürbis)-Kernöl, Malvenöl, Corylus Americana (Haselnuss)-Kernöl, Zea Mays (Mais)-Öl, Brassica Campestris (Raps)-Samenöl, Rapsöl, Sasanqua-Öl, Simmondsia Chinensis (Jojoba)-Kernöl, Rambutanöl, Sclerocarya Birrea Samenöl, Chenopodium Quinoa Samenöl oder Mischungen davon ausgewählt werden.

Das oder jedes pflanzliche Öl kann in den erfindungsgemäßen Haarbehandlungsmitteln bevorzugt in einer Menge von 0,01 bis 10 Gew.-%, mehr bevorzugt 0,05 bis 7,5 Gew.-% und besonders bevorzugt 0,1 bis 5 Gew.-% eingesetzt werden, wobei sich die Mengenangaben auf das Gesamtgewicht der Haarbehandlungsmittel beziehen.

Unter geeigneten Filmbildnern werden im Sinne der vorliegenden Erfindung filmbildende Polymere verstanden.

Unter Polymeren werden Makromoleküle mit einem Molekulargewicht von mindestens 1000 g/mol, bevorzugt von mindestens 2500 g/mol, besonders bevorzugt von mindestens 5000 g/mol, verstanden, welche aus gleichen sich wiederholenden organischen Einheiten bestehen. Bei den Polymeren der vorliegenden Erfindung kann es sich um synthetisch hergestellte Polymere handeln, die durch Polymerisation eines Monomertyps oder durch Polymerisation verschiedener, strukturell voneinander unterschiedlicher Monomertypen hergestellt werden. Wird das Polymer durch Polymerisation eines Monomertyps hergestellt, spricht man von Homo-Polymeren. Werden strukturell unterschiedliche Monomertypen in der Polymerisation eingesetzt, wird das resultierende Polymer als Copolymer bezeichnet.

Das maximale Molekulargewicht des Polymers hängt von dem Polymerisationsgrad (Anzahl der polymerisierten Monomere) und der Ansatzgröße ab und wird durch die Polymerisationsmethode mitbestimmt. Im Sinne der vorliegenden Erfindung ist es bevorzugt, wenn das maximale Molekulargewicht des filmbildenden Polymers nicht mehr als 10⁷ g/mol, bevorzugt nicht mehr als 10⁶ g/mol und besonders bevorzugt nicht mehr als 10⁵ g/mol beträgt.

Unter einem filmbildenden Polymer wird im Sinne der Erfindung ein Polymer verstanden, welches in der Lage ist, auf einem Substrat, beispielsweise auf einem keratinischen Material oder einer keratinischen Faser, einen Film auszubilden. Die Ausbildung eines Films kann beispielsweise durch Betrachtung des mit dem Polymer behandelten Keratinmaterials unter einem Mikroskop nachgewiesen werden.

Die filmbildenden Polymere können hydrophil oder hydrophob sein.

Im Rahmen einer ersten Ausführungsform kann es bevorzugt sein, mindestens ein hydrophobes, filmbildendes Polymer einzusetzen.

Unter einem hydrophoben Polymer wird ein Polymer verstanden, dass eine Löslichkeit in Wasser bei 25 °C (760 mmHg) von weniger als 1 Gew.-% besitzt.

Die Wasserlöslichkeit des filmbildenden, hydrophoben Polymers kann beispielsweise auf dem folgenden Weg bestimmt werden. 1,0 g des Polymers werden in ein Becherglas gegeben. Mit Wasser wird auf 100 g aufgefüllt. Es wird ein Rührfisch hinzugegeben, und die Mischung wird auf einem Magnetrührer unter Rühren auf 25 °C erwärmt. Es wird für 60 Minuten gerührt. Danach wird die wässrige Mischung visuell beurteilt. Sofern sich die Polymer-Wasser-Mischung aufgrund einer hohen Trübung des Gemisches nicht visuell beurteilten lässt, wird die Mischung filtriert. Bleibt auf dem Filterpapier ein Anteil an ungelöstem Polymer zurück, dann liegt die Löslichkeit des Polymers bei weniger als 1 Gew.-%.

Hier können insbesondere die Polymere des Acrylsäure-Typs, die Polyurethane, die Polyester, die Polyamide, die Polyharnstoffe, die Cellulose-Polymere, die Nitro-Cellulose-Polymere, die Silikon-Polymere, die Polymere des Acrylamid-Typs und die Polyisoprene genannt werden.

Besonders gut geeignete filmbildende, hydrophobe Polymere sind beispielsweise Polymere aus der Gruppe der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

In einer weiteren bevorzugten Ausführungsform ist ein erfindungsgemäßes Mittel dadurch gekennzeichnet, dass es mindestens ein filmbildendes, hydrophobes Polymer (c) enthält, das ausgewählt ist aus der Gruppe der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

Insbesondere die filmbildenden hydrophoben Polymere aus der Gruppe der synthetischen Polymere, die durch radikalische Polymerisation erhältlich sind oder natürliche Polymere sind besonders geeignet.

Weitere besonders gut geeignete filmbildende hydrophobe Polymere können ausgewählt werden aus den Homopolymere oder Copolymeren von Olefinen, wie beispielsweise Cycloolefinen, Butadien, Isopren oder Styren, Vinylethern, Vinylamiden, den Estern oder Amiden von (Meth)Acrylsäure mit mindestens einer C₁-C₂₀-Alkylgruppe, einer Arylgruppe oder einer C₂-C₁₀-Hydroxyalkylgruppe.

Weitere filmbildende hydrophobe Polymere können ausgewählt sein aus den Homo- oder Copolymeren von Isooctyl-(meth)acrylat; Isononyl-(meth)acrylat; 2-Ethylhexyl-(meth)acrylat; Lauryl-(meth)acrylat); Isopentyl-(meth)acrylat; n-Butyl-(meth)acrylat); Isobutyl-(meth)acrylat; Ethyl-(meth)acrylat; Methyl-(meth)acrylat; tert-Butyl-(meth)acrylat; Stearyl-(meth)acrylat; Hydroxyethyl-(meth)acrylat; 2-Hydroxypropyl-(methacrylat; 3-Hydroxypropyl-(meth)acrylat und/oder Gemischen hiervon.

Weitere filmbildende hydrophobe Polymere können ausgewählt sein aus den Homo- oder Copolymeren von (Meth)acrylamid; N-Alkyl-(meth)acrylamiden, insbesondere solchen mit C2-C18 Alkylgruppen, wie beispielsweise N-Ethyl-acrylamid, N-tert-butyl-acrylamid, le N-Octyl-crylamid; N-Di(C1-C4)alkyl-(meth)acrylamid.

Weitere bevorzugte anionische Copolymere sind beispielsweise Copolymere aus Acrylsäure, Methacrylsäure oder deren C₁-C₆-Alkylestern, wie sie unter der INCI-Deklaration Acrylates Copolymere vertrieben werden. Ein geeignetes Handelsprodukt ist beispielsweise Aculyn^{®} 33 der Firma Rohm & Haas. Weiterhin bevorzugt sind aber auch Copolymere aus Acrylsäure, Methacrylsäure oder deren C₁-C₆-Alkylestern und den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20.

Auf dem Markt befindliche ganz besonders bevorzugte Polymere sind beispielsweise Aculyn^{®}22 (Acrylates/Steareth-20 Methacrylate Copolymer), Aculyn^{®}28 (Acrylates/Beheneth-25 Methacrylate Copolymer), Structure 2001^{®}(Acryla-tes/Steareth-20 Itaconate Copolymer), Structure 3001^{®} (Acrylates/Ceteth-20 Itaconate Copolymer), Structure Plus^{®}(Acrylates/Aminoacrylates C10-30 Alkyl PEG-20 Itaconate Copolymer), Carbopol^{®} 1342, 1382, Ultrez 20, Ultrez 21 (Acrylates/C10-30 Alkyl Acrylate Crosspolymer), Synthalen W 2000^{®} (Acrylates/Palmeth-25 Acrylate Copolymer) oder das Rohm und Haas vertriebene Soltex OPT (Acrylates/C12-22 Alkyl methacrylate Copolymer). Als geeignete Polymere auf der Basis von Vinyl-Monomeren können beispielsweise genannt werden die Homo- und Copolymere des N-Vinylpyrrolidons, des Vinylcaprolactams, des Vinyl-(C1-C6-)alkyl-Pyrrols, des Vinyl-oxazols, des Vinyl-thiazols, des Vinylpyrimidins, des Vinylimidazols.

Weiterhin ganz besonders gut geeignet sind die Copolymere Octylacrylamid/acrylates/ butylaminoethyl-methacrylate copolymer, wie es beispielsweise unter den Handelsnamen AMPHOMER^{®} oder LOVOCRYL^{®} 47 von NATIONAL STARCH kommerziell vertrieben wird, oder auch die Copolymere von Acrylates/Octylacrylamide die unter den Handelsnamen DERMACRYL^{®} LT und DERMACRYL^{®} 79 von NATIONAL STARCH vertrieben werden.

Als geeignete Polymere auf der Basis von Olefinen können beispielsweise genannt werden die Homo- und Copolymere des Ethylens, des Propylens, des Butens, des Isoprens und des Butadiens. Im Rahmen einer weiteren Ausführungsform können als filmbildende hydrophobe Polymere die Block-Copolymere eingesetzt werden, die mindestens einen Block aus Styren oder den Derivaten des Styrens umfassen. Bei diesen Block-Copolymeren kann es sich um Copolymere handeln, die neben einem Styren-Block einen oder mehrere weitere Blöcke enthalten, wie beispielsweise Styren/Ethylen, Styren/Ethylen/Butylen, Styen/Butylen, Styren/Isopren, Styren/Butadien. Entsprechende Polymere werden von der BASF unter dem Handelsnamen "Luvitol HSB" kommerziell vertrieben.

Unter einem hydrophilen Polymer wird ein Polymer verstanden, dass eine Löslichkeit in Wasser bei 25 °C (760 mmHg) von mehr als 1 Gew.-%, bevorzugt von mehr als 2 Gew.-%, besitzt.

Die Wasserlöslichkeit des filmbildenden, hydrophilen Polymers kann beispielsweise auf dem folgenden Weg bestimmt werden. 1,0 g des Polymers werden in ein Becherglas gegeben. Mit Wasser wird auf 100 g aufgefüllt. Es wird ein Rührfisch hinzugegeben, und die Mischung wird auf einem Magnetrührer unter Rühren auf 25 °C erwärmt. Es wird für 60 Minuten gerührt. Danach wird die wässrige Mischung visuell beurteilt. Ein vollständig gelöstes Polymer erscheint makroskopisch homogen. Sofern sich die Polymer-Wasser-Mischung aufgrund einer hohen Trübung des Gemisches nicht visuell beurteilten lässt, wird die Mischung filtriert. Bleibt auf dem Filterpapier kein ungelöstes Polymer zurück, dann liegt die Löslichkeit des Polymers bei mehr als 1 Gew.-%.

Als filmbildende, hydrophile Polymere können nichtionische, anionische und kationische Polymere eingesetzt werden.

Geeignete filmbildende, hydrophile Polymere können beispielsweise aus der Gruppe der Polyvinylpyrrolidon-(Co)Polymere, der Polyvinylalkohol-(Co)Polymere, der Vinylacetat-(Co)Polymere, der Carboxyvinyl-(Co)Polymere, der Acrylsäure-(Co)Polymere, der Methacrylsäure-(Co)Polymere, der natürlichen Gummen, der Polysaccharide und/oder der Acrylamid-(Co)Polymere ausgewählt werden.

Besonders gut geeignete Polyvinylpyrrolidone sind beispielsweise unter der Bezeichnung Luviskol^{®} K von der BASF SE erhältlich, insbesondere Luviskol^{®} K 90 oder Luviskol^{®} K 85 der Firma BASF.

Als weiteres explizit ganz besonders gut geeignetes Polyvinylpyrrolidon (PVP) kann auch das Polymer PVP K30 eingesetzt werden, das von der Firma Ashland (ISP, POI Chemical) vertrieben wird. PVP K 30 ist ein in kaltem Wasser sehr gut lösliches Polyvinylpyrrolidon, welches die CAS-Nummer 9003-39-8 besitzt. Das Molgewicht von PVP K 30 liegt bei ca. 40000 g/mol.

Weitere ganz besonders gut geeignete Polyvinylpyrrolidone sind die unter den Handelsnamen LUVITEC K 17, LUVITEC K 30, LUVITEC K 60, LUVITEC K 80, LUVITEC K 85, LUVITEC K 90 und LUVITEC K 115 bekannten und von der BASF erhältlichen Substanzen.

Von den Vinylpyrrolidon-haltigen Copolymeren werden ganz besonders bevorzugt ein Styrene/VP Copolymer und/oder ein Vinylpyrrolidon-Vinylacetat-Copolymer und/oder ein VP/DMAPA Acrylates Copolymer und/oder ein VP/Vinyl Caprolactam/DMAPA Acrylates Copolymer in den kosmetischen Zusammensetzungen eingesetzt.

Vinylpyrrolidon-Vinylacetat-Copolymere werden unter der Bezeichnung Luviskol^{®} VA von der BASF SE vertrieben. Ein VP/Vinyl Caprolactam/DMAPA Acrylates Copolymer wird beispielsweise unter dem Handelsnamen Aquaflex^{®} SF-40 von Ashland Inc. vertrieben. Ein VP/DMAPA Acrylates Copolymer wird beispielsweise unter der Bezeichnung Styleze CC-10 von Ashland vertrieben und ist ein höchst bevorzugtes Vinylpyrrolidon-haltiges Copolymer.

Als weitere geeignete Copolymere des Polyvinylpyrrolidons können auch die Copolymere genannt werden, die durch Umsetzung von N-Vinylpyrrolidon mit mindestens einem weiteren Monomer aus der Gruppe aus V-Vinylformamid, Vinylacetat, Ethylen, Propylen, Acrylamid, Vinylcaprolactam, Vinylcaprolacton und/oder Vinylalkohol erhalten werden.

Kommen Copolymere aus N-Vinylpyrrolidon und Vinylacetat zum Einsatz, ist es wiederum bevorzugt, wenn das Molverhältnis der aus dem Monomer N-Vinylpyrrolidon enthaltenen Struktureinheiten zu den aus dem Monomer Vinylacetat enthaltenen Struktureinheiten des Polymers im Bereich von 20 zu 80 bis 80 zu 20, insbesondere von 30 zu 70 bis 60 zu 40, liegt. Geeignete Copolymerisate aus Vinylpyrrolidon und Vinylacetat sind beispielsweise unter dem Warenzeichen Luviskol^{®} VA 37, Luviskol^{®} VA 55, Luviskol^{®} VA 64 und Luviskol^{®} VA 73 von der Firme BASF SE erhältlich.

Ein weiteres besonders bevorzugtes Polymer wird dabei ausgewählt aus den Polymeren der INCI-Bezeichnung VP/Methacrylamide/Vinyl Imidazole Copolymer, die beispielsweise unter dem Handelsnamen Luviset Clear von der Fa. BASF SE erhältlich sind.

Ein weiteres ganz besonders bevorzugtes nichtionisches, filmbildendes, hydrophiles Polymer st ein Copolymer aus N-Vinylpyrrolidon und N,N-Dimethylaminiopropylmethacrylamid,welches beispielsweise mit der INCI-Bezeichnung VP/DMAPA Acrylates Copolymer z. B. unter der Handelsbezeichnung Styleze^{®} CC 10 von der Firma ISP verkauft wird.

Ein kationisches erfindungsgemäßes Polymer ist das Copolymer aus N-Vinylpyrrolidon, N-Vinylcaprolactam, N-(3-Dimethylaminopropyl)methacrylamid und 3-(Methacryloylamino)propyl-lauryl-dimethylammoniumchlorid (INCI-Bezeichnung: Polyquaternium-69), welches beispielsweise unter dem Handelsnamen AquaStyle^{®} 300 (28-32 Gew.-% Aktivsubstanz in Ethanol-WasserGemisch, Molekulargewicht 350000) von der Firma ISP vertrieben wird.

Weitere geeignete filmbildende, hydrophile Polymere sind beispielsweise
- Vinylpyrrolidon-Vinylimidazoliummethochlorid-Copolymere, wie sie unter den Bezeichnungen Luviquat^{®} FC 370, FC 550 und der INCI-Bezeichnung Polyquaternium-16 sowie FC 905 und HM 552 angeboten werden, - Vinylpyrrolidon-Vinylcaprolactam-Acrylat-Terpolymere, wie sie mit Acrylsäureestern und Acrylsäureamiden als dritter Monomerbaustein im Handel beispielsweise unter der Bezeichnung Aquaflex^{®} SF 40 angeboten werden.
- Polyquaternium-11 ist das Reaktionsprodukt von Diethylsulfat mit einem Copolymer von Vinylpyrrolidon und Dimethylaminoethylmethacrylat. Geeignete Handelsprodukte sind beispielsweise unter den Bezeichnungen Dehyquart^{®} CC 11 und Luviquat^{®} PQ 11 PN von der BASF SE oder Gafquat 440, Gafquat 734, Gafquat 755 oder Gafquat 755N von Ashland Inc. erhältlich.

- Polyquaternium-46 ist das Reaktionsprodukt von Vinylcaprolactam und Vinylpyrrolidon mit Methylvinylimidazoliummethosulfat und ist beispielsweise unter der Bezeichnung Luviquat^{®} Hold von der BASF SE erhältlich. Polyquaternium-46 wird bevorzugt in einer Menge von 1 bis 5 Gew.-% - bezogen auf das Gesamtgewicht der kosmetischen Zusammensetzung - eingesetzt. Es ganz besonders bevorzugt, dass Polyquaternium-46 in Kombination mit einer kationischen Guar-Verbindung eingesetzt wird. Es ist sogar höchst bevorzugt, dass Polyquaternium-46 in Kombination mit einer kationischen Guar-Verbindung und Polyquaternium-11 eingesetzt wird.

Als geeignete anionische filmbildende, hydrophile Polymere können zum Beispiel Acrylsäure-Polymere eingesetzt werden, die in unvernetzter oder vernetzter Form vorliegen können. Entsprechende Produkte werden beispielsweise unter den Handelsnamen Carbopol 980, 981, 954, 2984 and 5984 von der Firma Lubrizol oder auch unter den Namen Synthalen M and Synthalen K von der Firma 3V Sigma (The Sun Chemicals, Inter Harz) kommerziell vertrieben.

Geeignete filmbildende, hydrophile Polymere aus der Gruppe der Acrylamide sind beispielsweise Polymere, welche ausgehend von Monomeren der (Methy)Acrylamido-C1-C4-alkyl-sulfonsäure bzw. der Salze hiervon hergestellt werden. Entsprechende Polymere können ausgewählt sein aus den Polymeren von Polyacrylamidomethansulfonsäure, Polyacrylamidoethansulfonsäure, Polyacrylamidopropansulfonsäure, Poly2-acrylamido-2-methylpropansulfonsäure, Poly-2-Methylacrylamido-2-methylpropansulfonsäure und/oder Poly-2-methylacrylamido-n-butansulfonsäure.

Bevorzugte Polyemre der Poly(meth)arylamido-C1-C4-alkyl-sulfonsäuren sind vernetzt und zu mindestens 90 % neutralisiert. Diese Poylmere können vernetzt oder auch unvernetzt sein. Vernetzte und ganz oder teilweise neutraliserte Polymere des Typs der Poly-2-acrylamido-2-methylpropansulfonsäuren sind unter den INCI-Namen "Ammonium Polyacrylamido-2-methyl-propanesulphonate" oder "Ammonium Polyacryldimethyltauramide" bekannt.

Ein weiteres bevorzugtes Polymer dieses Typs ist das der Firma Clariant unter dem Handelsnamen Hostacerin AMPS vertriebene vernetzte Poly-2-acrylamido-2methyl-propanesulphonsäure-Polymer, das teilweise mit Ammoniak neutralisiert ist.

Das oder die filmbildende(n) Polymer(e) wird (werden) bevorzugt in bestimmten Mengenbereichen in den erfindungsgemäßen Haarbehandlungsmitteln eingesetzt. In diesem Zusammenhang hat es sich als besonders bevorzugt erwiesen, wenn die Haarbehandlungsmittel - bezogen auf ihr Gesamtgewicht - ein oder mehrere filmbildende Polymere in einer Gesamtmenge von 0,01 bis 10,0 Gew.-%, besonders bevorzugt von 0,05 bis 8,0 Gew.-%, und ganz besonders bevorzugt von 0,1 bis 7,5 Gew.-% enthalten.

Unter geeigneten Polyolen werden im Sinne der vorliegenden Anmeldung mehrwertige C2-C9-Alkanole mit zwei bis sechs Hydroxylgruppen und Polyethylenglycole mit 3 bis 20 EthylenoxidEinheiten verstanden.

Bevorzugt sind die C2-C10-Alkanole mit zwei bis acht Hydroxylgruppen ausgewählt aus 1,2-Propylenglycol, 1,3-Propylenglycol, 2-Methyl-1,3-propandiol, Glycerin, 1,2-Butylenglycol, 1,3-Butylenglycol, 1,4-Butylenglycol, 1,2-Pentandiol, 1,5-Pentandiol, 1,2-Hexandiol, 1,6-Hexandiol, 1,2,6-Hexantriol, 1,2-Octandiol, 1,8-Octandiol, Dipropylenglycol, Tripropylenglycol, Diglycerin, Triglycerin, Erythrit, Sorbit, cis-1,4-Dimethylolcyclohexan, trans-1,4-Dimethylolcyclohexan, beliebige Isomeren-Gemische von eis- und trans-1,4-Dimethylolcyclohexan sowie Mischungen dieser Polyole. Geeignete Polyethylenglycole sind vorzugsweise wasserlöslich und können ausgewählt sein aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind.

Weitere geeignete Polyole sind Methylolverbindungen, wie insbesondere Trimethylolethan, Trimethylolpropan, Trimethylolbutan, Pentaerythrit und Dipentaerythrit, Zuckeralkohole mit 5 bis 12 Kohlenstoffatomen, wie beispielsweise Sorbit oder Mannit, Zucker mit 5 bis 12 Kohlenstoffatomen wie beispielsweise Glucose oder Saccharose, oder Aminozucker wie beispielsweise Glucamin. Ganz besonders bevorzugte Polyole sind ausgewählt aus der Gruppe bestehend aus 1,2-Propylenglycol, Glycerin, 1,3-Butylenglycol, PEG-3, PEG-4, PEG-5, PEG-6, PEG-7, PEG-8, Caprylyl Glycol (1,2-Octandiol) und Mischungen daraus.

Insbesondere bevorzugte Polyole sind 1,2-Propylenglycol, 1,3-Butylenglycol, Glycerin, Caprylyl Glycol oder Mischungen davon.

Polyole können in den erfindungsgemäßen Haarbehandlungsmitteln vorzugsweise in Mengen von 0,1 bis 20,0 Gew.-%, besonders bevorzugt von 0,25 bis 15,0 Gew.-% und insbesondere bevorzugt von 0,5 bis 10,0 Gew.-% eingesetzt werden, wobei sich die Mengenangaben auf das Gesamtgewicht der Haarbehandlungsmittel beziehen.

Als geeignete Fettalkohole können eingesetzt werden gesättigte, ein- oder mehrfach ungesättigte, verzweigte oder unverzweigte Fettalkohole mit 6 bis 30, bevorzugt 10 bis 30 und ganz besonders bevorzugt 14 bis 24 Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll.

In einer bevorzugten Ausführungsform sind die Fettalkohole aus natürlichen Fettsäuren erhältlich, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden.

Die Einsatzmenge von Fettalkoholen in den erfindungsgemäßen Haarpflegemitteln beträgt bevorzugt 0,1 - 20 Gew.-%, bezogen auf das gesamte Mittel. Besonders bevorzugt beträgt die Einsatzmenge von Fettalkoholen in den erfindungsgemäßen Haarpflegemitteln 0,5 - 15 Gew.-%, wobei ganz besonders vorteilhaft Mengen von 1,0 - 10 Gew.-% sein können.

In einer besonders bevorzugten Ausführungsform enthalten die erfindungsgemäßen Haarpflegemittel zur Optimierung der Rheologieeigenschaften mindestens einen gesättigten oder ungesättigten, verzweigten oder unverzweigten Fettalkohol mit 10 bis 24 Kohlenstoffatomen in einer Menge von 1,0 bis 10,0 Gew.-% (bezogen auf das Gewicht des gesamten Mittels).

Innerhalb dieser Ausführungsform ist es besonders bevorzugt, wenn die erfindungsgemäßen Haarpflegemittel Cetylalkohol, Stearylalkohol, Gemische von Cetylalkohol und Stearylalkohol (Cetearylalkohol), Arachidylalkohol, Behenylalkohol oder Mischungen davon in den zuvor genannten Mengen enthalten.

Als kationische Tenside eignen sich in erfindungsgemäßen Haarbehandlungsmitteln prinzipiell alle für die Verwendung am menschlichen Körper geeigneten kationischen oberflächenaktiven Stoffe. Diese sind gekennzeichnet durch mindestens eine wasserlöslich machende, kationische Gruppe, wie z. B. eine quaternäre Ammonium-Gruppe, oder durch mindestens eine wasserlöslich machende, kationisierbare Gruppe, wie z. B. eine Amin-Gruppe und weiterhin mindestens eine lipophile Alkylgruppe mit etwa 6 bis 30 C-Atomen, oder auch durch mindestens eine Imidazol-Gruppe oder mindestens eine Imidazylalkyl-Gruppe.

Im Allgemeinen werden katonische Tenside je nach ihren Strukturmerkmalen in Gruppen aufgeteilt. Besonders geeignet für die Verwendung in den erfindungsgemäßen Zusammensetzungen sind kationische Tenside d) aus mindestens einer der Gruppen der
i. Alkylquats,
ii. Esterquats,
iii. quarternären Imidazolinen,
iv. Amidoaminen und/oder kationisierten Amidoaminen,
v. und/oder Mischungen davon.

Diese spezifisch benannten kationischen Tenside haben in den erfindungsgemäßen Haarbehandlungsmitteln eine als besonders angenehm empfundenen Konditionierwirkung gezeigt. Besonders bevorzugte erfindungsgemäße Haarbehandlungsmittel enthalten als kationische Tenside d) quarternäre Ammoniumverbindungen (Alkylquats), Amidoamine, kationisierte Amidoamine sowie Mischungen hiervon.

Ganz besonders bevorzugte quaternäre Ammoniumverbindungen (Alkylquats) weisen mindestens einem C₈-C₂₄-Alkylrest auf und sind ausgewählt aus quaternären Ammoniumhalogeniden, insbesondere Chloriden, oder aus quaternären Ammoniumalkylsulfaten, wie Methosulfaten oder Ethosulfaten, beispielsweise C₈-C₂₄-Alkyltrimethylammoniumchloride, C₈-C₂₄-Dialkyldimethylammoniumchloride und C₈-C₂₄-Trialkylmethylammoniumchloride wie Cetyltrimethylammoniumchlorid, Stearyltrimethylammoniumchlorid, Behentrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27, Quaternium-83, Quaternium-87 und Quaternium-91 bekannten Imidazolium-Verbindungen.

Bei Esterquats handelt es sich um kationische Tenside, die sowohl mindestens eine Esterfunktion als auch mindestens eine quartäre Ammoniumgruppe als Strukturelement und weiterhin mindestens einen C8-C24-Alkylrest oder C8-C24-Acylrest enthalten. Bevorzugte Esterquats sind quaternierte Estersalze von Fettsäuren mit Triethanolamin, quaternierte Estersalze von Fettsäuren mit Diethanolalkylaminen und quaternierte Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. N,N-Bis(2-Palmitoyloxyethyl)dimethylammoniumchlorid, Distearoylethyl Dimonium Methosulfate und Distearoylethyl Hydroxyethylmonium Methosulfate sind bevorzugte Beispiele für solche Esterquats. Alkylamidoamine werden üblicherweise durch Amidierung natürlicher oder synthetischer C₈-C₂₄-Fettsäuren und Fettsäureschnitte mit Di-(C₁-C₃)alkylaminoaminen hergestellt. Erfindungsgemäß besonders geeignete Verbindungen aus dieser Substanzgruppe sind beispielsweise unter den INCI-Bezeichnungen Brassicamidopropyldimethylamin, Lauramidopropyldimethylamin, Myristamidopropyldimethylamin, Stearamidopropyldimethylamin, Cocamidopropyldimethylamin, Ricinolamidopropyldimethylamin, Isostearamidopropyldimethylamin, Oleamidopropyldimethylamin, Behenamidopropyldimethylamin und/oder Palmamidopropyldimethylamin bekannte Verbindungen. Insbesondere bevorzugt ist Stearamidopropyl Dimethylamine.

Unter kationisierten Amidoaminen werden vorzugsweise unter den INCI-Bezeichnungen Behenamidopropylethyldimoniumethosulfat, Behenamidopropyl-PG-dimoniumchlorid, Oleamidopropylethyldimoniumethosulfat, Oleamidopropyl-PG-dimoniumchlorid, Cocamidopropylethyldimoniumethosulfat, Cocamidopropyltrimoniumchlorid, Ricinoleamidopropylethyldimoniumethosulfat, Rinoleamidopropyltrimoniumchlorid, Ricinoleamidopropyltrimoniummethosulfat, Stearamidopropylethyldimoniumethosulfat, Stearamidopropyltrimoniummethosulfat, Undecylenamidopropyltrimoniummethosulfat, Lauramidopropyl-PG-dimoniumchlorid und/oder Canolamidopropylethyldimoniumethosulfat, bekannte Verbindungen verstanden.

Kationische Tenside können in den erfindungsgemäßen Haarbehandlungsmitteln vorzugsweise in Mengen von 0,1 bis 10 Gew.-%, besonders bevorzugt 0,25 bis 7,5 Gew.-% und 0,5 bis 5 Gew.-% eingesetzt werden (bezogen auf das Gesamtgewicht der Haarbehandlungsmittel).

Trimethylglycin (INCI-Bezeichnung: Betaine) ist ein für die Verwendung in kosmetischen Mitteln wohlbekanntes Aminosäurederivat, das aus natürlichen Quellen wie aus Melasse der Zuckerrübe erhältlich ist. Es wird im Handel beispielsweise von der Firma Evonik unter der Bezeichnung Tego^{®} Natural Betaine angeboten und findet Anwendung als Feuchthaltemittel in Präparaten für die Hautbehandlung sowie als festigender Wirkstoff in Haarbehandlungsmitteln.

Trimethylglycin kann in den erfindungsgemäßen Haarbehandlungsmitteln in einem Gewichtsanteil von 0,05 bis 2,5 Gew.-% am Gesamtgewicht der Haarbehandlungsmittel eingesetzt werden. Einsatzmengen von 0,1 bis 2,0 Gew.-% und insbesondere von 0,25 bis 1,5 Gew.-% sind bevorzugt. Es wurde beobachtet, dass die haarpflegenden Eigenschaften der bisher thematisierten Inhaltsstoffe durch Hinzufügen von Trimethylglycin weiter verbessert werden können. Speziell der weiche Haargriff bis in die Haarspitzen lässt sich durch die Kombination der drei Wirkstoffe verbessern. Darüber hinaus kann dadurch die Problematik statisch aufgeladener Haare erheblich reduziert werden. Besonders ausgeprägt lassen sich diese vorteilhaften Wirkungen bei einem Arbeiten in den zuvor definierten Konzentrationsbereichen erzielen.

In einer bevorzugten Ausführungsform handelt es sich bei dem erfindungsgemäßen Haarbehandlungsmittel um ein Styling-Gel.

In einer weiteren bevorzugten Ausführungsform handelt es sich bei dem erfindungsgemäßen Haarbehandlungsmittel um eine Combing Cream oder um ein Haarbalsam.

Ein zweiter Gegenstand der Erfindung ist die Verwendung des erfindungsgemäßen Haarbehandlungsmittels in Leave-on-Produkten, insbesondere in Leave-on-Produkten zur Verbesserung der Frisierbarkeit.

Ein dritter Gegenstand der Erfindung ist ein Verfahren zur Haarbehandlung, bei dem ein erfindungsgemäßes kosmetisches Mittel auf die nassen oder trockenen Haare aufgetragen, einmassiert oder verteilt und vorzugsweise bis zur nächsten Haarreinigung auf den Haaren belassen wird.

Bevorzugt sind Haarbehandlungsverfahren, die der Frisurgebung oder dem Styling von Haaren dienen.

Die Erfindung kann durch die folgenden nummerierten Aussagen gekennzeichnet sein:
1. Haarbehandlungsmittel, umfassend in einem kosmetischen Träger
   a) 3-Hydroxypropyloctansäureester (Propandiol Caprylate) und
   b) mindestens ein Wachs.
2. Haarbehandlungsmittel nach Aussage 1, wobei der Gewichtsanteil des oder jedes 3-Hydroxypropyloctansäureesters (Propandiol Caprylate) 0,01 - 5 Gew.-% am Gesamtgewicht des Haarbehandlungsmittels beträgt.
3. Haarbehandlungsmittel nach einer der vorhergehenden Aussagen, wobei das oder jedes Wachs ein natürliches Wachs ist.
4. Haarbehandlungsmittel nach einer der vorhergehenden Aussagen, wobei das oder jedes Wachs ein pflanzliches oder tierisches Wachs ist.
5. Haarbehandlungsmittel nach einer der vorhergehenden Aussagen, wobei das oder jedes Wachs aus Candelillawachs, Carnaubawachs, Espartograswachs, Korkwachs, Zuckerrohrwachs, Ouricurywachs, Sonnenblumen(kern)wachs, Jojobawachs, Rapswachs, Sojawachs, Reiswachs, Rosenblütenwachs, Fruchtwachs, Myrica Fruchtwachs, Bienenwachs, Insektenwachs, Walrat, Wollwachs, Bürzelfett, Shea Butter, Kakaobutter oder Mischungen davon ausgewählt ist.
6. Haarbehandlungsmittel nach einer der vorhergehenden Aussagen, wobei das oder jedes Wachs aus Candelillawachs (Euphorbia Cerifera (Candelilla) Wax), Carnaubawachs (Copernicia Cerifera (Carnauba) Wax), Beerenwachs (Rhus Verniciflua Peel Cera), Apfel(schalen)wachs (Pyrus Malus (Apple Peel) Wax), Orangen(schalen)wachs (Citrus Aurantium Dulcis Peel Wax) oder Mischungen davon ausgewählt ist.
7. Haarbehandlungsmittel nach einer der vorhergehenden Aussagen, wobei der Gewichtsanteil des oder jedes Wachses 0,001 - 20 Gew.-% am Gesamtgewicht des Haarbehandlungsmittels beträgt.
8. Haarbehandlungsmittel nach einer der vorhergehenden Aussagen, umfassend zusätzlich mindestens ein natürliches Verdickungsmittel.
9. Haarbehandlungsmittel nach einer der vorhergehenden Aussagen, umfassend zusätzlich mindestens ein natürliches Verdickungsmittel aus der Gruppe der Gumme.
10. Haarbehandlungsmittel nach einer der Aussagen 8 oder 9, wobei der Gewichtsanteil des oder jedes Gummis (Gums) 0,001 - 10 Gew.-% am Gesamtgewicht des Haarbehandlungsmittels beträgt.
11. Haarbehandlungsmittel nach einer der Aussagen 8 bis 10, wobei das oder jedes Gummi ausgewählt ist aus Caesalpinia Spinosa Gum, Xanthan Gum oder Mischungen davon.
12. Haarbehandlungsmittel nach einer der vorhergehenden Aussagen, umfassend - bezogen auf das Gesamtgewicht des Haarbehandlungsmittels -
   a) 0,01 - 5 Gew.-% Hydroxypropyloctansäureesters (Propandiol Caprylate),
   b) 0,001 - 20 Gew.-% Candelillawachs (Euphorbia Cerifera (Candelilla) Wax), Carnaubawachs (Copernicia Cerifera (Carnauba) Wax), Beerenwachs (Rhus Verniciflua Peel Cera), Apfel(schalen)wachs (Pyrus Malus (Apple Peel) Wax), Orangen(schalen)wachs (Citrus Aurantium Dulcis Peel Wax) oder Mischungen davon.
13. Haarbehandlungsmittel nach einer der vorhergehenden Aussagen, umfassend - bezogen auf das Gesamtgewicht des Haarbehandlungsmittels -
   a) 0,01 - 5 Gew.-% Hydroxypropyloctansäureesters (Propandiol Caprylate),
   b) 0,001 - 20 Gew.-% Candelillawachs (Euphorbia Cerifera (Candelilla) Wax), Carnaubawachs (Copernicia Cerifera (Carnauba) Wax), Beerenwachs (Rhus Verniciflua Peel Cera), Apfel(schalen)wachs (Pyrus Malus (Apple Peel) Wax), Orangen(schalen)wachs (Citrus Aurantium Dulcis Peel Wax) oder Mischungen davon,
   c) 0,001 - 10 Gew.-% Caesalpinia Spinosa Gum, Xanthan Gum oder Mischungen davon.
14. Haarbehandlungsmittel nach einer der vorhergehenden Aussagen, enthaltend zusätzlich mindestens ein pflanzliches Öl in einem Gewichtsanteil von 0,01 - 10 Gew.-% am Gesamtgewicht des Haarbehandlungsmittels.
15. Haarbehandlungsmittel nach einer der vorhergehenden Aussagen, enthaltend zusätzlich mindestens einen Filmbildner in einem Gewichtsanteil von 0,01 -10 Gew.-% am Gesamtgewicht des Haarbehandlungsmittels.
16. Haarbehandlungsmittel nach einer der vorhergehenden Aussagen, enthaltend zusätzlich mindestens ein Polyol in einem Gewichtsanteil von 0,01 - 20 Gew.-% am Gesamtgewicht des Haarbehandlungsmittels.
17. Haarbehandlungsmittel nach einer der vorhergehenden Aussagen, enthaltend Glycerin, 1,2-Propandiol, Caprylyl Glycol, 1,3-Butylenglycol oder Mischungen davon.
18. Haarbehandlungsmittel nach einer der vorhergehenden Aussagen, enthaltend zusätzlich mindestens einen Fettalkohol in einem Gewichtsanteil von 0,1 - 20 Gew.-% am Gesamtgewicht des Haarbehandlungsmittels.
19. Haarbehandlungsmittel nach einer der vorhergehenden Aussagen, enthaltend Cetylalkohol, Stearylalkohol, Gemische von Cetylalkohol und Stearylalkohol (Cetearylalkohol), Arachidylalkohol, Behenylalkohol oder Mischungen davon.
20. Haarbehandlungsmittel nach einer der vorhergehenden Aussagen, enthaltend zusätzlich mindestens ein kationisches Tensid in einem Gewichtsanteil von 0,1 - 10 Gew.-% am Gesamtgewicht des Haarbehandlungsmittels.
21. Haarbehandlungsmittel nach einer der vorhergehenden Aussagen, enthaltend zusätzlich mindestens ein kationisches Tensid in einem Gewichtsanteil von 0,05 - 2,5 Gew.-% am Gesamtgewicht des Haarbehandlungsmittels.
22. Haarbehandlungsmittel nach einer der vorhergehenden Aussagen in Form eines Stylinggels.
23. Haarbehandlungsmittel nach einer der Aussagen 1 bis 21 in Form einer Combing Cream oder eines Haarbalsams.
24. Verwendung eines kosmetischen Haarbehandlungsmittels nach einer der Aussagen 1 bis 23 in leave-on Produkten, insbesondere in leave-on Produkten zur Verbesserung der Frisierbarkeit.
25. Verfahren zur Haarbehandlung, bei dem ein kosmetisches Mittel nach einer der Aussagen 1 bis 23 auf die nassen oder trockenen Haare aufgetragen, einmassiert oder verteilt und vorzugsweise bis zur nächsten Haarreinigung auf den Haaren belassen wird.
26. Verfahren nach Aussage 25, wobei das Verfahren zur Haarbehandlung ein Verfahren zur Frisurgebung oder ein Stylingverfahren ist.

### Beispiele

Alle angegebenen Mengen betreffen Gewichtsteile. Die folgenden Formulierungen wurden unter Verwendung bekannter Herstellungsverfahren bereitgestellt.

| | **Beispiel 1** | **Beispiel 2** | **Beispiel 3** | **Beispiel 4** |
|---|---|---|---|---|
| **Propandiol Caprylate** | 0,1 - 1,0 | 0,1 - 1,0 | 0,1 - 1,0 | 0,1 - 1,0 |
| **Pflanzenwachs** | 0,01 - 10 | 0,01 - 10 | 0,01 - 10 | 0,01 - 10 |
| **natürliches Verdickungsmittel** | | 0,001 - 10 | | |
| **Filmbildner** | | | 0,01 - 10 | |
| **Polyol** | | | 0,01 - 20 | 0,01 - 20 |
| **Wasser und ggfs. weitere Hilfs- und Zusatzstoffe** | ad 100 | ad 100 | ad 100 | ad 100 |

| | **Beispiel 4** | **Beispiel 5** | **Beispiel 6** | **Beispiel 7** |
|---|---|---|---|---|
| **Propandiol Caprylate** | 0,1 - 1,0 | 0,1 - 1,0 | 0,1 - 1,0 | 0,1 - 1,0 |
| **Candelillawachs, Carnaubawachs, Beerenwachs, Apfel(schalen)wachs und/oder Orangen(schalen)wachs** | 0,01 - 10 | 0,01 - 10 | 0,01 - 10 | 0,01 - 10 |
| **Hydroxypropyl Starch** | 4,0 - 7,0 | | 4,0 - 7,0 | 4,0 - 7,0 |
| **Dehydroxanthan Gum** | | 0,5 - 4,0 | | |
| **Acrylates/Steareth-20 Methylacrylate Crosspolymer** | | | 0,5 - 2,5 | 0,5 - 2,5 |
| **Glycerin** | | | | 0,1 - 2,0 |
| **Wasser und ggfs. weitere Hilfs- und Zusatzstoffe** | ad 100 | ad 100 | | |

Bei den Beispielen 3 - 7 handelt es sich um Haargele.

| | **Beispiel 9** | **Beispiel 10** | **Beispiel 11** | **Beispiel 12** |
|---|---|---|---|---|
| **Propandiol Caprylate** | 0,1 - 1,0 | 0,1 - 1,0 | 0,1 - 1,0 | 0,1 - 1,0 |
| **Pflanzenwachs** | 0,01 - 10 | 0,01 - 10 | 0,01 - 10 | 0,01 - 10 |
| **natürliches Verdickungsmittel** | | 0,001 - 10 | 0,001 -10 | 0,001 -10 |
| **Fettalkohol** | | | 2,0 - 10,0 | |
| **Polyol** | | | | 0,01 - 20 |
| **pflanzliches Öl und/oder Emollient** | 0-1,5 | 0-1,5 | 0-1,5 | 0-1,5 |
| **kationisches Tensid** | 0-1,5 | 0-1,5 | 0-1,5 | 0-1,5 |
| **Wasser und ggfs. weitere Hilfs- und Zusatzstoffe** | ad 100 | ad 100 | ad 100 | ad 100 |

| | **Beispiel 13** | **Beispiel 14** | **Beispiel 15** | **Beispiel 16** |
|---|---|---|---|---|
| **Propandiol Caprylate** | 0,1 - 1,0 | 0,1 - 1,0 | 0,1 - 1,0 | 0,1 - 1,0 |
| **Candelillawachs, Carnaubawachs, Beerenwachs, Apfel(schalen)wachs und/oder Orangen(schalen)wachs** | 0,01 - 10 | 0,01 - 10 | 0,01 - 10 | 0,01 - 10 |
| **Caesalpinia Spinosa Gum** | 0,05 - 0,5 | | 0,05 - 0,5 | |
| **Dehydroxanthan Gum** | | 0,5 - 4,0 | | 0,5 - 4,0 |
| **Cetearyl Alcohol, Arachiodyl Alcohol, Behenyl Alcohol** | | 2,0 - 10,0 | 2,0 - 10,0 | 2,0 - 10,0 |
| **Glycerin** | 0 - 5,0 | 0 - 5,0 | 1,0 - 5,0 | 1,0 - 5,0 |
| **Distearoylethyl Dimonium Chloride** | 0-1,5 | 0-1,5 | 0,5 - 1,5 | 0,5 - 1,5 |
| **Betaine** | 0-1,5 | 0-1,5 | 0,5 - 1,5 | 0,5 - 1,5 |
| **Wasser und ggfs. weitere Hilfs- und Zusatzstoffe** | ad 100 | ad 100 | ad 100 | ad 100 |

Bei den Beispielen 11 - 16 handelt es sich um Combing Creams bzw. Haarbalsame.

Die beispielhaften Haarbehandlungsmittel verleihen damit behandelten Haaren ein gesundes, gepflegtes Erscheinungsbild und eine angenehme Haptik. Insbesondere lassen sich Haare nach der Behandlung sehr gut frisieren und weisen ein gepflegtes und geschmeidiges Haargefühl bis in die Haarspitzen sowie strahlenden Glanz auf.

Es versteht sich, dass die Erfindung im Rahmen der beigefügten Patentansprüche modifiziert werden kann.

## Patentansprüche

1. Haarbehandlungsmittel, umfassend in einem kosmetischen Träger
a. 3-Hydroxypropyloctansäureester (Propandiol Caprylate) und
b. mindestens ein Wachs.

2. Haarbehandlungsmittel nach Anspruch 1, wobei der Gewichtsanteil des oder jedes 3-Hydroxypropyloctansäureesters (Propandiol Caprylate) 0,01 - 5 Gew.-% am Gesamtgewicht des Haarbehandlungsmittels beträgt.

3. Haarbehandlungsmittel nach einem der vorhergehenden Ansprüche, wobei das oder jedes Wachs ein natürliches Wachs ist.

4. Haarbehandlungsmittel nach einem der vorhergehenden Ansprüche, wobei das oder jedes Wachs aus Candelillawachs (Euphorbia Cerifera (Candelilla) Wax), Carnaubawachs (Copernicia Cerifera (Carnauba) Wax), Beerenwachs (Rhus Verniciflua Peel Cera), Apfel(schalen)wachs (Pyrus Malus (Apple Peel) Wax), Orangen(schalen)wachs (Citrus Aurantium Dulcis Peel Wax) oder Mischungen davon ausgewählt ist.

5. Haarbehandlungsmittel nach einem der vorhergehenden Ansprüche, wobei der Gewichtsanteil des oder jedes Wachses 0,001 - 20 Gew.-% am Gesamtgewicht des Haarbehandlungsmittels beträgt.

6. Haarbehandlungsmittel nach einem der vorhergehenden Ansprüche, umfassend zusätzlich mindestens ein natürliches Verdickungsmittel.

7. Haarbehandlungsmittel nach Anspruch 6, umfassend mindestens ein natürliches Verdickungsmittel aus der Gruppe der Gumme.

8. Haarbehandlungsmittel nach einem der Ansprüche 6 oder 7, wobei das oder jedes Gummi ausgewählt ist aus Caesalpinia Spinosa Gum, Xanthan Gum oder Mischungen davon.

9. Haarbehandlungsmittel nach einem der Ansprüche 6 bis 8, wobei der Gewichtsanteil des oder jedes Gummis (Gums) 0,001 - 10 Gew.-% am Gesamtgewicht des Haarbehandlungsmittels beträgt.

10. Haarbehandlungsmittel nach einem der vorhergehenden Ansprüche, umfassend - bezogen auf das Gesamtgewicht des Haarbehandlungsmittels -
a) 0,01 - 5 Gew.-% Hydroxypropyloctansäureester (Propandiol Caprylate),
b) 0,001 - 20 Gew.-% Candelillawachs (Euphorbia Cerifera (Candelilla) Wax), Carnaubawachs (Copernicia Cerifera (Carnauba) Wax), Beerenwachs (Rhus Verniciflua Peel Cera), Apfel(schalen)wachs (Pyrus Malus (Apple Peel) Wax), Orangen(schalen)wachs (Citrus Aurantium Dulcis Peel Wax) oder Mischungen davon,
c) 0,001 - 10 Gew.-% Caesalpinia Spinosa Gum, Xanthan Gum oder Mischungen davon.

11. Haarbehandlungsmittel nach einem der vorhergehenden Ansprüche, enthaltend zusätzlich mindestens ein pflanzliches Öl in einem Gewichtsanteil von 0,01 - 10 Gew.-% am Gesamtgewicht des Haarbehandlungsmittels.

12. Haarbehandlungsmittel nach einem der vorhergehenden Ansprüche, enthaltend zusätzlich mindestens einen Filmbildner in einem Gewichtsanteil von 0,01 -10 Gew.-% am Gesamtgewicht des Haarbehandlungsmittels.

13. Haarbehandlungsmittel nach einem der vorhergehenden Ansprüche, enthaltend zusätzlich mindestens ein Polyol in einem Gewichtsanteil von 0,01 - 20 Gew.-% am Gesamtgewicht des Haarbehandlungsmittels.

14. Verwendung eines kosmetischen Haarbehandlungsmittels nach einem der Ansprüche 1 bis 13 in Leave-on Produkten, insbesondere in Leave-on Produkten zur Verbesserung der Frisierbarkeit.

15. Verfahren zur Haarbehandlung, bei dem ein kosmetisches Mittel nach einem der Ansprüche 1 bis 13 auf die nassen oder trockenen Haare aufgetragen, einmassiert oder verteilt und vorzugsweise bis zur nächsten Haarreinigung auf den Haaren belassen wird.
